# EUROPEAN PATENT APPLICATION

(11) **EP 4 393 446 A1**
(43) Date of publication of application: **03.07.2024**
(21) Application number: 22216747.0
(22) Date of filing: 27.12.2022
(51) Int. Cl.: A61B 90/40, B25J 21/00, B01L 1/00

(54) **AN ISOLATOR MODULE FOR ROBOTIC ARM**

(71) Applicant: Uimei Srls, 24060 Tavernola Bergamasca (BG) (IT)
(72) Inventor: FORESTI, Ruben, 43125 PARMA (IT)
(74) Representative: Dondi, Silvia

(57) **Abstract**

An isolator module (1) for robotic arm, comprising:
a box-like casing (2) defining a working chamber (3) isolated from an external environment;
means (4) for realizing a controlled contamination environment in said chamber (3);
said box-like casing (2) comprising at least one opening (7),
said module (1) comprising a glove (8) intended to house the robotic arm,
said glove (8) being hermetically mounted at said at least one opening (7) such that an external surface thereof is exposed to the working chamber (3) and an internal surface thereof is communicating with the external environment,
said glove (8) comprising:
- a positioning system (9) for positioning the robotic arm inside the glove (8);
- one or more terminals (10) for the interaction of the robotic arm with the chamber (3).

## Description

The present invention relates to an isolator module for a robotic arm.

The invention finds application in the management of aseptic development processes.

It is known to use robots inside isolators.

The main drawback linked to this solution is that the isolators must be made ad hoc since much larger dimensions than the standard ones are required in order to accommodate the robots therein. In particular, these isolators are about four times larger than normal. This leads to an increase in the structural complexity, as well as increased costs and greater difficulty in keeping such a large environment decontaminated.

There is therefore a need for an alternative solution that allows a robot to carry out works inside the isolator.

In this context, the technical task underlying the present invention is to provide an isolator module for robotic arm, which obviates the drawbacks in the prior art as described above.

In particular, the object of the present invention is to provide an isolator module for robotic arm, wherein a robotic arm can carry out works within the isolated environment, but at the same time whose dimensions are not bulky. Another object of the present invention is to make available an isolator module for robotic arm, which is versatile.

The specified technical task and the specified purposes are substantially achieved by an isolator module for robotic arm, comprising:
a box-like casing defining a working chamber isolated from an external environment;
creation means for creating a controlled contamination environment in said chamber;
said box-like casing comprising at least one opening.

The module comprises a glove intended to house the robotic arm, said glove being hermetically mounted at said at least one opening such that an external surface thereof is exposed to the working chamber and an internal surface thereof is communicating with the external environment.

The glove comprises:
- a positioning system for positioning the robotic arm inside the glove;
- one or more terminals for the interaction of the robotic arm with the camera. Preferably, the positioning system is of the magnetic or electromagnetic type.

In accordance with one embodiment, the positioning system comprises a plurality of metal plates distributed along the glove.

Preferably, the metal plates are located on the internal surface of the glove. In accordance with one embodiment, the isolator module comprises signalling means for signalling the position of the robotic arm with respect to the box-like casing.

Preferably, the glove comprises an arm portion and an end portion, said metal plates being distributed along the arm portion.

In accordance with one embodiment, the box-like casing comprises support means for working tools inside the chamber.

In accordance with one embodiment, the isolator module comprises a recognition system for recognizing the position of the robotic arm with respect to the support means.

In accordance with one embodiment, the support means comprises a rack and the isolator module comprises one or more tools mounted on the rack. The recognition system comprises one or more notches obtained on the rack and/or on the tool and a notch detection sensor. The sensor is mounted on the glove.

In accordance with one embodiment, the isolator module comprises at least one attachment for a cooling module or a heating module or for a drawer incubator.

Preferably, the attachment is obtained on a hatch mounted on a rear portion of the box-like casing.

In accordance with one embodiment, the isolator module comprises a passage compartment obtained laterally on the box-like casing for the transfer of materials.

In accordance with an embodiment, the terminals may comprise: isolation means and/or gripping and actuation means and/or viewing means and/or cutting means and/or heating means and/or electromagnetic means.

In accordance with one embodiment, the glove comprises at least one wetting channel and at least one drying channel such that it is intended for washing.

In accordance with one embodiment, the glove comprises piezoelectrics or load cells.

In accordance with one embodiment, the glove comprises material welding devices.

In accordance with one embodiment, the glove comprises mechanical couplings intended for the use of medical instrumentations such as laparoscopic devices.

Further features and advantages of the present invention will become more apparent from the approximate and thus non-limiting description of a preferred, but non-exclusive, embodiment of an isolator module for robotic arm, as illustrated in the accompanying drawings, wherein:
- figure 1 illustrates an isolator module for robotic arm, according to the present invention, in a schematic view from the back;
- figure 2 illustrates the isolator module in a schematic side view;

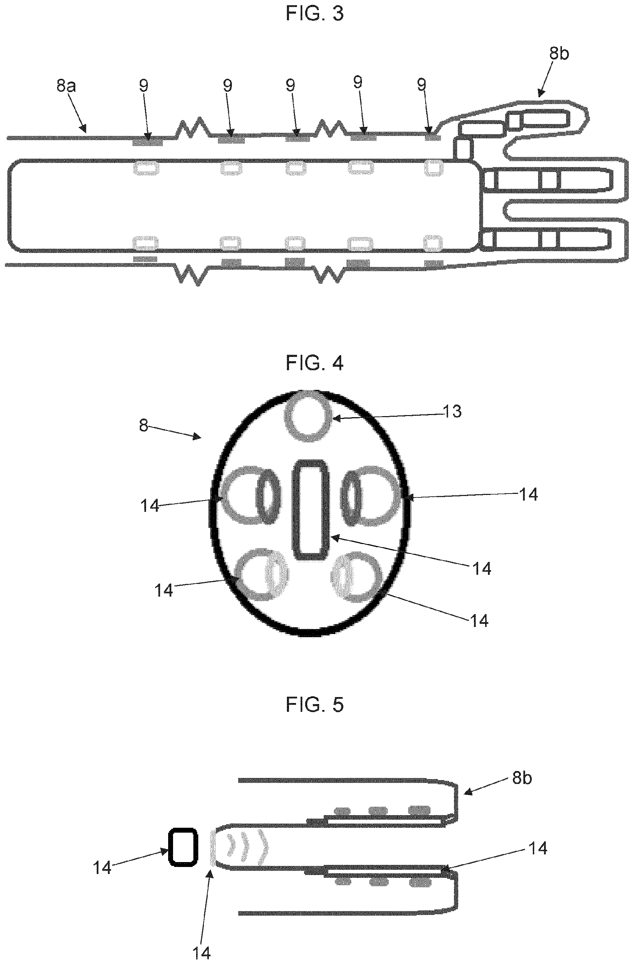

- figure 3 illustrates a schematic view of a detail (glove) of the isolator module of figure 2, with a robotic arm therein;
- figure 4 illustrates a schematic view of a section of the glove of figure 2;
- figure 5 illustrates a schematic view of an end portion of the glove of figure 2.

With reference to the figures, numeral 1 denotes an isolator module for robotic arm.

The module 1 comprises a box-like casing 2 defining a working chamber 3 isolated from an external environment. The external environment is for example a room in which module 1 is located.

The module 1 comprises means 4 for realizing a controlled contamination environment in the chamber 3. Controlled contamination environment means an aseptic environment. Preferably, the means 4 comprises a sanitizing unit 5. For example, the sanitizing unit 5 comprises a system for the distribution of steam of H₂O₂ or VHP in the chamber 3.

Preferably, the means 4 comprises a maintenance unit 6 for maintaining the predefined environmental conditions. The maintenance unit 6 comprises humidity management means and UV lamps.

Preferably, at least the sanitizing unit 5 is arranged above the box-like casing 2.

Preferably, the box-like casing 2 comprises at least one opening 7. The module 1 comprises a glove 8 hermetically mounted at the opening 7 such that an external surface thereof is exposed to the working chamber 3 and an internal surface thereof is communicating with the external environment. In this way, an external operator, such as a robotic arm, can interact from the outside in the working chamber 3 without compromising the controlled contamination environment that is established.

By hermetically mounting the glove 8 on the opening 7 it is meant that there is no exchange of fluids between the external environment and the chamber 3 through the opening 7.

In the preferred embodiment, the box-like casing 2 comprises two openings 7 and two corresponding gloves 8.

Originally, the glove 8 comprises a positioning system 9 for positioning the robotic arm therein. In this way, the self-centring of the robotic arm inside the glove 8 is ensured.

Preferably, the positioning system 9 is of the magnetic or electromagnetic type.

In the embodiment described and illustrated herein, the positioning system 9 comprises a plurality of metal plates (indicated with the same reference numeral 9) distributed along the glove 8. The robotic arm will have corresponding magnetic portions for recognizing the correct position inside the glove 8.

Preferably, the metal plates 9 are distributed on the internal surface of the glove 8.

Preferably, the glove 8 comprises an arm portion 8a and an end portion 8b. Preferably, the end portion 8b is shaped to house an end effector of the robotic arm. The end effector is the device at the end of a robotic arm, designed to interact with the environment.

Preferably, the metal plates 9 affect the arm portion 8a.

Preferably, the glove 8 comprises a quick coupling system. Preferably, the glove 8 comprises an air decompression system between the glove 8 and the robotic arm.

Originally, the glove 8 comprises one or more terminals 10 for the interaction of the robotic arm with the chamber 3. In particular, the terminals 10 are chosen according to the operations that the robotic arm must perform inside the chamber 3. Below is an exemplary and non-exhaustive list of the possible terminals 10 that may be employed. Any terminal 10 that allows a robotic arm to perform a known operation inside chamber 3 is to be considered implicitly included.

Among the terminals 10, by way of example it is possible to identify isolation means, gripping and actuation means, viewing means, cutting means, heating means, electromagnetic means, etc. For example, the glove 8 may comprise a terminal 10 for gripping a drill.

Among the terminals 10 there is also preferably a glass for an optical device (chamber) of the robotic arm.

Among the terminals 10, an infrared probe is preferably present.

Among the terminals 10, pressure and temperature sensors are preferably present.

Preferably, the terminals 10 are mounted inside the glove 8. Preferably, the terminals 10 are welded on the glove 8.

Preferably, the box-like casing 2 comprises support means 11 for working tools inside the chamber 3. Preferably, the support means 11 comprise a rack (indicated by the same reference numeral 11).

Preferably, the isolator module 1 comprises a recognition system for recognizing the position of the robotic arm with respect to the support means 11.

In one embodiment, the recognition system comprises one or more notches or grooves 12 obtained on the rack 11 and a detection sensor 13 for detecting the groove12. The sensor 13 is mounted on the glove 8. In this way, the robotic arm knows where to find the tool.

Preferably, the sensor 13 is of the infrared or contact type.

In one embodiment, the isolator module 1 comprises one or more tools 18 mounted on the support means 11. The notches 12 are made on the tools 18.

In one embodiment, the notches 12 are made on both the tools 18 and on the rack 11.

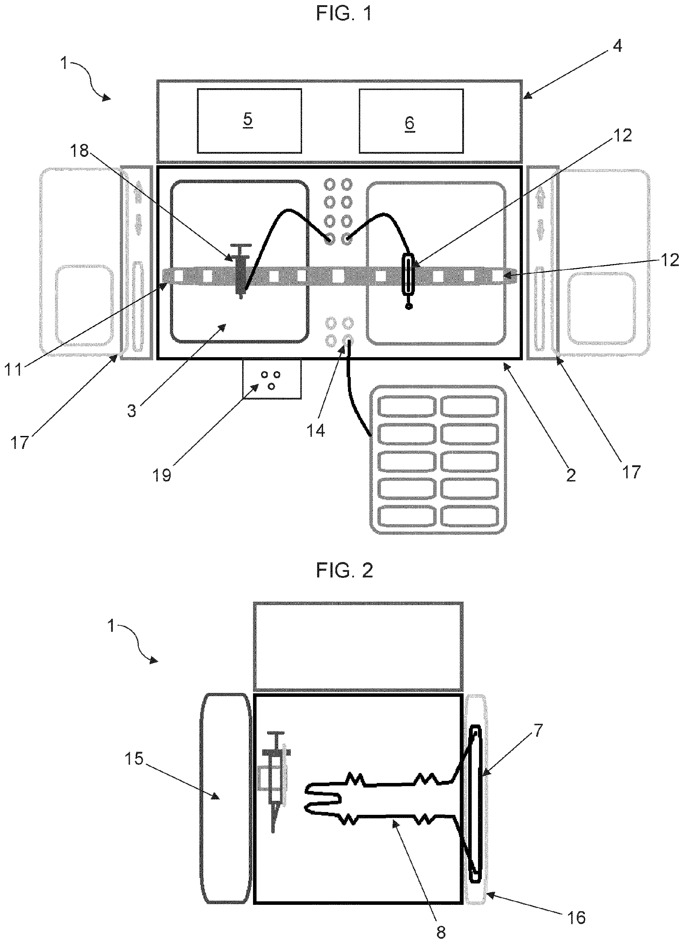

Suitably, the isolator module 1 may comprise signalling means 19 for signalling the position of the robotic arm with respect to the box-like casing 2, in particular with respect to the opening 7. For example, as illustrated in figure 1, the signalling means 19 comprises one or more holes with preestablished dimensions and geometries. These are used for the spatial centring of the robotic arm, which can therefore be placed on an automated guided vehicle (AGV).

Preferably, the box-like casing 2 comprises at least one attachment 14 for a cooling module or a heating module or for a drawer incubator. In this way, the isolator module 1 can be configured as an isolator, climatic chamber or incubator, as desired.

Preferably, the attachment 14 is obtained on a hatch 15 mounted on a rear portion (the so-called back) of the box-like casing 2.

Preferably, the box-like casing 2 comprises a front screen 16 to allow viewing the chamber 3 from the outside. Preferably, the opening 7 is obtained on the front screen 16.

Preferably, the isolator module 1 comprises a passage compartment 17 for the transfer of materials (managed inside the chamber 3) between modules. The passage compartment 17 is obtained laterally on the box-like casing 2. In accordance with one embodiment, the glove 8 comprises at least one wetting channel and at least one drying channel such that it is intended for washing.

In accordance with one embodiment, the glove 8 comprises piezoelectrics or load cells.

In accordance with one embodiment, the glove 8 comprises material welding devices.

In accordance with one embodiment, the glove 8 comprises mechanical couplings intended for the use of medical instrumentations such as laparoscopic devices.

An isolator module 1 can be assembled in sequence with various modules to create an isolator system that meets production needs.

The modules that make up this isolator system are placed side by side and connected laterally.

Suitably, a material transfer module is connected to the side of the first module of the row (i.e. upstream) and/or to the side of the last module of the row (i.e. downstream). This module is of known type and will not be further described. In particular, this module comprises bio-decontamination means. Its function is in fact to allow the entry or the exit of materials with respect to the working chamber 3 of the adjacent module 1 without compromising the aseptic condition of the material. In particular, it comprises a vertical sliding door with inflatable seals, magnetic and assisted closure.

In accordance with one embodiment, the isolator system comprises four modules 1 connected in sequence.

The first module 1 is provided with HEPA H14 filter and is kept at a temperature comprised between +80 °C and -100 °C. The first module 1 is provided with a glove 8.

The second module 1 is kept at a temperature comprised between +80 °C and -100 °C. The second module 1 is provided with two gloves 8.

The third module 1 is kept at a temperature comprised between +50 °C and -5 °C and a CO₂ control is carried out.

The fourth module 1 acts as an incubator and is kept at a temperature of +37 °C with CO₂ control.

Between adjacent modules 1 there are connection modules or exchange compartments.

In the preferred embodiment, the isolator system comprises three isolator units. Each unit comprises one or more modules 1 as described above.

A first unit is used for the preparation of work materials. Preferably, the first unit comprises two modules 1 connected to each other and a material transfer module on the side of the last module 1, i.e. downstream.

The first module 1 is used for sanitizing the material. The second module 1 is used for preparing the material.

A second unit is used for the processing and post-processing of materials. Preferably, the second unit comprises two modules 1 connected to each other and a material transfer module on the side of the last module 1, i.e. downstream.

In the modules 1 of the second unit, a humidity and temperature control is carried out to keep a temperature range comprised between -100 °C and 150 °C.

In particular, the first module 1 of the second unit is connected upstream of the material transfer module of the first unit.

The first module 1 of the second unit houses a 3D printer or thermoforming tools.

The second module 1 of the second unit is used for preparing the material for the post-development treatment.

Preferably, one or more automated arms for manipulation at extreme temperatures are provided inside one or more modules 1 of the second unit. The material transfer module of the second unit is used both for loading post-processing materials and those for cell cultures.

A third unit is used for incubation and cell cultures. Preferably, the third unit comprises three modules 1 connected to each other and a material transfer module on the side of the last module 1, i.e. downstream.

In the modules 1 of the third unit, a CO₂, O₂ and temperature control is carried out to set environmental conditions suitable for the cultures.

The first module 1 of the third unit is used for the preparation of media and cells.

The second module 1 of the third unit comprises a rack to store the tests for growth (to increase the exploitation of the useful area)

The third module 1 of the third unit comprises a temperature-controlled microscope for analysis and digital data collection.

The characteristics of the isolator module for robotic arm, according to the present invention, are clear from the description, as are the advantages.

In particular, the presence on the glove of a positioning system for positioning the robotic arm inside the glove and of one or more terminals for the interaction of the robotic arm with the chamber allow to use a robotic arm without introducing it inside the isolator module. This allows the dimensions of the isolator to be kept small and consequently the surface to be sanitized.

In addition, the possibility of connecting thermal modules (heating or freezing) or a drawer incubator makes the isolator module versatile, which can act as an isolator, climatic chamber or incubator. The presence of attachments for these modules/incubator on the back of the box-like casing allows to convey a function to a surface which in the known solutions is not used.

## Claims

1. An isolator module (1) for robotic arm, comprising:
a box-like casing (2) defining a working chamber (3) isolated from an external environment;
means (4) for realizing a controlled contamination environment in said chamber (3);
said box-like casing (2) comprising at least one opening (7),
said module (1) comprising a glove (8) intended to house the robotic arm,
said glove (8) being hermetically mounted at said at least one opening (7) such that an external surface thereof is exposed to the working chamber (3) and an internal surface thereof is communicating with the external environment,
said glove (8) comprising:
- a positioning system (9) for positioning the robotic arm inside the glove (8);
- one or more terminals (10) for the interaction of the robotic arm with the chamber (3).

2. The isolator module (1) according to claim 1, wherein the positioning system (9) is of the magnetic or electromagnetic type.

3. The isolator module (1) according to claim 2, wherein the positioning system (9) comprises a plurality of metal plates (9) distributed along the glove (8).

4. The isolator module (1) according to claim 3, wherein the metal plates (9) are located on the internal surface of the glove (8).

5. The isolator module (1) according to any one of the preceding claims, comprising signalling means (18) for signalling the position of the robotic arm with respect to the box-like casing (2).

6. The isolator module (1) according to claim 3 or 4 or 5, wherein said glove (8) comprises an arm portion (8a) and an end portion (8b), said metal plates (9) being distributed along the arm portion (8a).

7. The module (1) according to any one of the preceding claims, wherein said box-like casing (2) comprises support means (11) for working tools (18) inside the chamber (3).

8. The isolator module (1) according to claim 7, comprising a recognition system (12, 13) for recognizing the position of the robotic arm with respect to the support means (11).

9. The isolator module (1) according to claim 8, wherein the support means (11) comprises a rack (11), said isolator module (1) comprising one or more tools (18) mounted on the rack (11), said recognition system (12, 13) comprising one or more notches (12) obtained on the rack (11) and/or on the tool (18) and a detection sensor (13) for detecting the notch (12), said sensor (13) being mounted on the glove (8).

10. The isolator module (1) according to any one of the preceding claims, comprising at least one attachment (14) for a cooling module or a heating module or for a drawer incubator.

11. The isolator module (1) according to claim 10, wherein said at least one attachment (14) is obtained on a hatch (15) mounted on a rear portion of the box-like casing (2).

12. The isolator module (1) according to any one of the preceding claims, comprising a passage compartment (17) obtained laterally on the box-like casing (2) for the transfer of materials.

13. The isolator module (1) according to any one of the preceding claims, wherein the terminals (10) may comprise: isolation means and/or gripping and actuation means and/or viewing means and/or cutting means and/or heating means and/or electromagnetic means.

14. The isolator module (1) according to any one of the preceding claims, wherein said at least one glove (8) comprises at least one wetting channel and at least one drying channel such that it is intended for washing or it comprises piezoelectrics or load cells or it comprises material welding devices.

15. The isolator module (1) according to any one of the preceding claims, wherein said at least one glove (8) comprises mechanical couplings intended for the use of medical instrumentations such as laparoscopic devices.
